# EUROPEAN PATENT APPLICATION

(11) **EP 0 812 841 A1**
(43) Date of publication of application: **17.12.1997**
(21) Application number: 97109666.4
(22) Date of filing: 13.06.1997
(51) Int. Cl.: C07D 471/04, A61K 31/47

(54) **Crystal of N-((quinolin-2-yl)-phenyl)sulfonamides and process for producing the same**

(30) Priority: 13.06.1996 JP 152464/96
(71) Applicant: ASAHI GLASS COMPANY LTD., Chiyoda-ku, Tokyo (JP); THE GREEN CROSS CORPORATION, Osaka-shi Osaka (JP)
(72) Inventor: Okazoe, Takashi, c/o Asahi Glass Co., Ltd., Yokohama-shi, Kanagwa (JP); Morizawa, Yoshitomi, c/o Asahi Glass Co., Ltd., Yokohama-shi, Kanagwa (JP)
(74) Representative: Hansen, Bernd, Dr. Dipl.-Chem.

(57) **Abstract**

A crystal of an N-[(quinolin-2-yl)phenyl]sulfonamide represented by the following formula (1) or a crystal of a salt thereof: 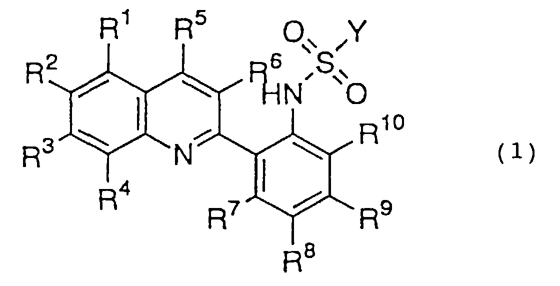 the substituents are defined in the specification is described. The crystal is useful as a pressor pharmaceutical compound. A process for producing the crystal is also described.

## Description

### FIELD OF THE INVENTION

The present invention relates to crystals of N-[(quinolin-2-yl)phenyl]sulfonamides or salts thereof which are angiotensin II antagonists with great potential as a remedy for hypertension and a process for producing the same.

### BACKGROUND OF THE INVENTION

Blood pressure of a living organism is regulated by the sympathetic nervous system, the balance between the pressor system and the depressor system, and the like. The reninangiotensin system participates in the pressor system. Renin acts on angiotensinogen to form angiotensin I. Angiotensin I is converted into angiotensin II by angiotensin I converting enzyme. Angiotensin II is a potent vasoconstrictor and acts on the adrenal cortex to promote the secretion of aldosterone, thereby causing an increase in the blood pressure. Angiotensin II exerts its function via an angiotensin II receptor on cell membranes. Therefore, an antagonist to angiotensin II is effective as a remedy for hypertension mediated by angiotensin II, in a fashion similar to that of an angiotensin I converting enzyme inhibitor.

There have been reported non-peptide angiotensin II antagonists which can be orally administered (JP-A-56-71074, JW-A-3-501020, WO 93/19060, and the like; the terms "JP-A" and "JW-A" as used herein mean an unexamined published Japanese patent application" and an "unexamined published Japanese patent application based on a PCT application", respectively). The present inventors have conducted extensive studies to find non-peptide compounds having angiotensin II antagonism and being efficacious when administered orally. As a result, they have successfully found out that quinoline derivatives are effective therefor (JP-A-6-16659, JP-A-6-80664). Among these, N-[(quinolin-2-yl)phenyl]sulfonamides and salts thereof are highly expected to be useful as a remedy for hypertension because of their excellent absorption characteristics *in vivo*.

To produce N-[(quinolin-2-yl)phenyl]sulfonamides and salts thereof, it has been a practice by the present inventors that a (quinolin-2-yl)aniline is allowed to react with trifluoromethanesulfonic anhydride in a chlorinated solvent in the presence of a base and, after work-up, the reaction product is purified by silica gel column chromatography with ethyl acetate/chloroform as an eluent. Then the target product is obtained by evaporating to dryness without performing crystallization.

Although the target product thus obtained, which is in the form of a solid, is inherently an amorphous one, the X-ray diffraction pattern thereof indicates that it has a crystallinity of a certain degree. Also, a large amount of chloroform remains in the solid product thus obtained. When the solution containing the target compound is spray-dried, on the other hand, the product thus obtained is not a completely amorphous solid but shows some crystallinity. The stability of the solid product obtained by spray-drying is examined while varying conditions such as temperature and humidity. Thus it is found out that this product undergoes further crystallization under certain conditions. Namely, it is revealed that this amorphous product has a poor stability as amorphous.

### SUMMARY OF THE INVENTION

The present invention relates to crystals of an N-[(quinolin-2-yl)phenyl]sulfonamide and salts thereof which are expected to be useful as a remedy for hypertension and a process for producing the crystal, as will be described hereinbelow. The crystal of the present invention has a high purity and an extremely high stability in being a crystal. The process of the present invention makes it possible to efficiently and easily produce the crystal. High purity crystals include crystals suitable for pharmaceutical use. These crystals may be stable crystals wherein crystallization would not proceed by usual operations.

That is, the present invention provides a crystal of an N-[(quinolin-2-yl)phenyl]sulfonamide represented by the following formula (1) or a crystal of a salt thereof: 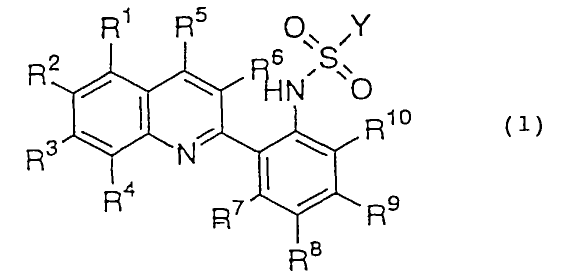
wherein R¹, R³, R⁴, R⁵, R⁶, R⁷, R⁸, R⁹ and R¹⁰ each independently represents a hydrogen atom, a halogen atom, a lower alkyl group, a cyclo lower alkyl group, an aryl group, an aralkyl group, an alkoxy group or -CₘF₂ₘ₊₁;
R² represents a hydrogen atom, a halogen atom, a lower alkyl group, a cyclo lower alkyl group, an aryl group, an aralkyl group, an alkoxy group, -CₙF₂ₙ₊₁ or -CH₂Q, in which Q represents a halogen atom or a monovalent organic group bonded to the carbon atom of -CH₂Q via a nitrogen atom of Q;
Y represents -CₓF₂ₓ₊₁ or an aryl group; and
m, n and x each independently represents an integer of from 1 to 6.

Furthermore, the present invention provides a process for producing a crystal of an N-[(quinolin-2-yl)phenyl]sulfonamide or a crystal of a salt thereof which comprises crystallizing a crystal of an N-[(quinolin-2-yl)phenyl]sulfonamide represented by formula (1) or a crystal of a salt thereof from a solvent solution containing an alcohol.

### BRIEF DESCRIPTION OF THE DRAWINGS

Fig. 1 shows the X-ray diffraction spectrum of the crystals obtained in Example 1.

Fig. 2 shows the X-ray diffraction spectrum of the crystals obtained in Example 3.

Fig. 3 shows the X-ray- diffraction spectrum of the crystals obtained in Example 4.

Fig. 4 shows the X-ray diffraction spectrum of the product obtained in Reference Example 4.

### DETAILED DESCRIPTION OF THE INVENTION

The N-[(quinolin-2-yl)phenyl]sulfonamides represented by formula (1) are publicly known compounds fundamentally and described in JP-A-6-16659 and JP-A-6-80664 relating to the inventions of the present inventors as described above. First, the compounds represented by formula (1) will be described.

The term "lower" organic group as used herein means one having 1 to 6 carbon atoms.

Examples of the lower alkyl group include a methyl group, an ethyl group, a propyl group, an isopropyl group, a butyl group, an isobutyl group, a t-butyl group, a pentyl group and a hexyl group.

The alkenyl group is preferably a lower alkenyl group, and more preferably an alkenyl group having 2 to 4 carbon atoms. Examples thereof include a vinyl group, an allyl group, a 1-propenyl group and a 1-butenyl group.

The term "cyclo lower alkenyl group" means a cycloalkyl group having 3 to 6 carbon atoms in its ring. Examples thereof include a cyclopropyl group, a cyclobutyl group, a cyclopentyl group and a cyclohexyl group.

The term "halogen atom" means a fluorine atom, a chlorine atom, a bromine atom and an iodine atom.

Examples of the halo lower alkyl group include a chloromethyl group, a 2-chloroethyl group, a 1,2-dichloroethyl group and a 3-trifluoropropyl group.

The term "aryl group means a monovalent aromatic hydrocarbon group. A phenyl group and derivatives thereof are preferred. Examples thereof include a phenyl group, a tolyl group and a p-halophenyl group.

The term "aralkyl group" means an aryl-substituted alkyl group. The alkyl group preferably has not more than 4 carbon atoms. Examples thereof include a benzyl group, a benzhydryl group, a trityl group and a phenethyl group.

The alkoxy group is preferably a lower alkoxy group, and more preferably an alkoxy group having not more than 4 carbon atoms. Examples thereof include a methoxy group, an ethoxy group, a propoxy group and a butoxy group.

The alkoxy lower alkyl group is preferably a lower alkyl group having a lower alkoxy group. Examples thereof include a methoxyethyl group, a 3-methoxypropyl group and a 2-ethoxyethyl group.

The alkylthio group is preferably a lower alkylthio group. Examples thereof include a methylthio group, an ethylthio group and a butylthio group.

R¹, R², R³, R⁴ and R⁵ are each preferably a hydrogen atom, a lower alkyl group or an aryl group, and more preferably a hydrogen atom or a lower alkyl group having not more than 4 carbon atoms. In addition, R² is preferably -CH₂Q. At the same time, R⁶, R⁷, R⁸ and R⁹ are each preferably a hydrogen atom, a halogen atom or a lower alkyl group. Preferred examples of the halogen atom include a chlorine atom and a fluorine atom.

R² is more preferably a hydrogen atom, a methyl group or -CH₂Q, and most preferably -CH₂Q. As a compound having a pharmaceutically preferred physiological activity, namely, it is particularly preferred that R² is -CH₂Q having an imidazole ring, an imidazopyridine ring, and the like as described in the above-mentioned patent applications. In such a case, it is preferred that R¹ to R⁵ excluding R² are a hydrogen atom.

R⁶, R⁷, R⁸ and R⁹ are each preferably a hydrogen atom, or at least one of them is a halogen atom (in particular, a chlorine atom) while others are hydrogen atoms.

Q represents a monovalent organic group bonded to the carbon atom of -CH₂Q via a nitrogen atom of Q. The monovalent organic group is obtained by eliminating from an organic compound having an -NH- group the hydrogen atom bonded to the nitrogen atom. The organic compound having an -NH- group is preferably a heterocyclic compound wherein the nitrogen atom in the -NH- group constitutes the ring. This heterocyclic compound may be a fused ring heterocyclic compound. The organic compound having an -NH- group may be an aliphatic amine compound, an alicyclic amine compound or an aromatic amine compound each having at least one primary or secondary amino group.

Preferred examples of Q include the residues of heterocyclic compounds, namely, the residues of substituted imidazole compounds as described in the above-mentioned patents (those obtained by eliminating the hydrogen atom bonded to the nitrogen atom constituting the heteroring) and the residues of substituted imidazopyridine compounds (those obtained by eliminating the hydrogen atom bonded to the nitrogen atom constituting the heteroring). That is to say, it is preferred to use as Q, for example, a substituted 1H-imidazol-1-yl group represented by the following formula (2), more preferably a substituted 3H-imidazo[4,5-b]pyridin-3-yl group represented by the following formula (3):

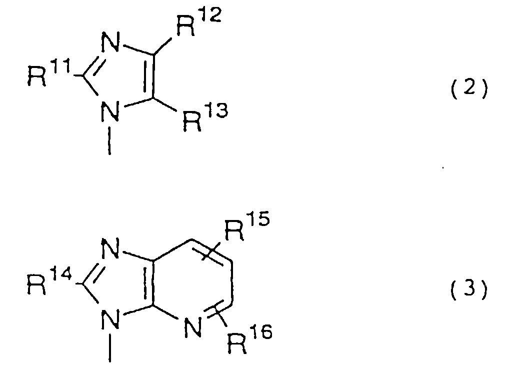

In the above formulae (2) and (3), R¹¹ to R¹⁶ are defined below.

R¹¹ and R¹⁴ each independently represents a lower alkyl group, a halo lower alkyl group, a cyclo lower alkyl group, an alkenyl group, an alkoxy group, an alkoxy lower alkyl group or an alkylthio group.

R¹² and R¹³ are the same or different and each independently represents a hydrogen atom, a halogen atom, -CᵢF₂ᵢ₊₁, -(CH₂)ₚR²⁰, -(CH₂)ᵣCOR²¹, or -(CH₂)ₜNR²²COR²³.

R¹⁵ and R¹⁶ are the same or different and each independently represents a hydrogen atom, a halogen atom, a lower alkyl group, a halo lower alkyl group, a cyclo lower alkyl group, an alkenyl group, an alkoxy group, -CⱼF₂ⱼ₊₁, -(CH₂)_{q}R²⁴, or -(CH₂)ₛCOR²⁵.

In the above definitions, R²⁰ to R²⁵ and i to t have the following meanings.

R²⁰ and R²⁴ each independently represents a hydroxyl group or an alkoxy group.

R²¹ and R²⁵ each independently represents a hydrogen atom, a lower alkyl group or an alkoxy group.

R²² represents a hydrogen atom or a lower alkyl group.

R²³ represents a hydrogen atom, a lower alkyl group or an alkoxy group.

i and j each independently represents an integer of from 1 to 6.

p and q each independently represents an integer of from 1 to 4.

r and s each independently represents an integer of from 0 to 4.

t represents an integer of from 0 to 4.

It is preferred that R¹¹-and R¹⁴ are each a lower alkyl group; R¹² is a chlorine atom; R¹³ is -(CH₂)ₚR²⁰ (wherein R²⁰ represents a hydroxyl group; and p is 1), or -(CH₂)ᵣCOR²¹ (wherein R²¹ represents a hydrogen atom or a lower alkoxy group; and r is 0 or 1); and R¹⁵ and R¹⁶ are the same or different and are each a hydrogen atom, a lower alkyl group, -(CH₂)_{q}R²⁴ (wherein R²⁴ represents a hydroxyl group; and q is 1), or -(CH₂)ₛCOR²⁵ (wherein R²⁵ represents a hydrogen atom or a lower alkoxy group; and s is 0 or 1).

In the compounds represented by formula (1), Y represents -CₓF₂ₓ₊₁ or an aryl group. Y is preferably -CₓF₂ₓ₊₁, wherein x is from 1 to 4, or a phenyl group, or more preferably a trifluoromethyl group. In -CₘF₂ₘ₊₁ and -CₙF₂ₙ₊₁, m and n are each preferably from 1 to 4, and more preferably 1.

Salts of N-[(quinolin-2-yl)phenyl]sulfonamides include acid addition salts of these compounds with inorganic or organic acids. As these acids, use may be made of pharmaceutically acceptable acids. Examples of the salts include hydrochlorides, hydrobromides, sulfates, phosphates, methanesulfonates, p-toluenesulfonates, oxalates, tartarates, citrates, maleates, fumarates, succinates, lactates, glutarates, acetates, trifluoroacetates and salts of various amino acids.

In the present invention, the N-[(quinolin-2-yl)phenyl]sulfonamides may be usually obtained by reacting (quinolin-2-yl)anilines represented by the following formula (4) with sulfonic acids represented by Y-SO₃H or reactive derivatives thereof in a solvent in `he presence of a base, though the present invention is not restricted thereto:

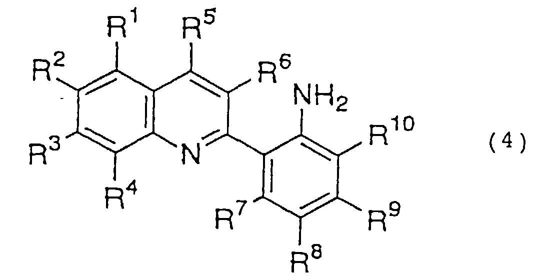

The reactive derivatives of the sulfonic acids include acid anhydrides, acid halides, and the like. It is particularly preferred to use acid anhydrides such as trifluoromethanesulfonic anhydride therefor. As the reaction solvent, use may be made of those in which the reactants and the products are soluble. It is particularly appropriate to use chlorinated solvents such as dichloromethane, chloroform and 1,2-dichloroethane therefor. As the base, it is preferred to use organic bases such as triethylamine and pyridine.

The most preferred example of the N-[(quinolin-2-yl)phenyl]sulfonamides in the present invention is N-{2-{6-[(2-ethyl-5,7-dimethyl-3H-imidazo[4,5-b]pyridin-3-yl)methyl] quinolin-2-yl}phenyl}trifluoromethanesulfonamide.

As described above, N-{2-{6-[(2-ethyl-5,7-dimethyl-3H-imidazo[4,5-b]pyridin-3-yl)methyl]quinolin-2-yl}phenyl}trifluoromethanesulfonamide can be obtained by reacting 2-{6-[(2-ethyl-5,7-dimethyl-3H-imidazo[4,5-b]pyridin-3-yl)methyl]quinolin-2-yl}aniline with trifluoromethanesulfonic anhydride in a solvent in the presence of a base. Trifluoromethanesulfonic anhydride is employed in an amount of 0.5 to 10 equivalents, preferably 1 to 2 equivalents, to the aniline. As described above, it is preferred to use a chlorinated solvent as the solvent, while an organic base is preferred as the base. The base may be preferably employed in an amount of 1 to 5 equivalents to trifluoromethanesulfonic anhydride. The reaction temperature typically ranges from -78 to +30°C, preferably from -20 to +20°C. The reaction time typically ranges from 0.1 to 10 hours, preferably from 0.5 to 5 hours.

The process for producing crystals according to the present invention is characterized in that crystals of an N-[(quinolin-2-yl)phenyl]sulfonamide or a salt thereof are produced by crystallizing a crude of N-[(quinolin-2-yl)phenyl]sulfonamide or a salt thereof from a soluble solvent solution containing an alcohol. The term "crude" means the target product which has not been sufficiently purified. Examples thereof include a solution containing the reaction product formed by the above reaction, a substance obtained by concentrating the solution, one obtained by evaporating the solution to dryness and one obtained by partly purifying (for example, washing) the reaction product in the form of a solution or a solid. The meaning of the term "crystallization" as used herein may involve recrystallization of crystals once formed for purification, and the like.

The N-[(quinolin-2-yl)phenyl]sulfonamides or salts thereof are sparingly soluble compounds and, therefore, sufficiently soluble exclusively in chlorinated solvents among common solvents, when employed alone. Other solvents in which these compounds are sufficiently soluble are mixtures of alcohols with hydrocarbon solvents or ester solvents. Also, these compounds are soluble in alcohols containing hydrogen chloride, and the like. Accordingly, these solvent systems can be used for the crystallization. However, it is preferred that the final solvent to be used is one free from any chlorinated solvent, when the medical acceptability is taken into consideration.

Examples of the solvents to be used together with alcohols so as to obtain a solvent system include chlorinated solvents, hydrocarbon solvents, ester solvents and ketone solvents, though the present invention is not restricted thereto. It is particularly preferred to use chlorinated solvents such as dichloromethane, chloroform and 1,2-dichloroethane, hydrocarbon solvents such as toluene or ether solvents such as ethyl acetate therefor. The solvent may be one employed as the reaction solvent.

As the alcohol, it is preferred to use alkanols having 1 to 4 carbon atoms such as methanol, ethanol, 2-propanol and 1-butanol. Ethanol is the most preferred. In the soluble solvent containing an alcohol,-the ratio by volume of the alcohol to the other solvent typically ranges from 0.1 to 20, preferably from 0.2 to 2, although the present invention is not restricted thereto.

It is also possible that hydrogen halides such as hydrogen chloride or other acids as cited above are used together with alcohols and the resulting acid-containing alcohols are employed as the soluble solvent for producing crystals. Use of these acid-containing alcohols makes it possible to form N-[(quinolin-2-yl)phenyl]sulfonamide salts. When N-[(quinolin-2-yl)phenyl]sulfonamides are dissolved in hydrogen chloride-containing alcohols, for example, crystals of N-[(quinolin-2-yl)phenyl]sulfonamide hydrochlorides can be obtained.

The crystallization can be performed by methods known in the art.

Subsequently, the crystals thus formed can be separated from the solvent and the residual solvent is removed by drying. Thus powdery crystals of the target product can be obtained. Drying is preferably carried out at 60°C or higher under reduced pressure. The crystals obtained by the process of the present invention are highly pure and extremely stable, compared with those obtained by the convectional methods.

It is also possible in some cases to perform recrystallization so as to give crystals with an elevated purity. As the solvent for the recrystallization, it is preferred to employ those other than chlorinated solvents, since they are more pharmaceutically acceptable. Other than alcohols, it is preferred to use ester solvents such as ethyl acetate or hydrocarbon solvents such as toluene therefor. The crystals are dissolved in a mixture solvent comprising this solvent with alcohols. Alternatively, recrystallization can be effected by using a mixture of hydrogen chloride/alcohol as the solvent. The most preferred solvent for the recrystallization is a mixture of ethyl acetate/ethanol.

A preferred example of the process for producing crystals will be described.

In a chlorinated solvent in the presence of a base, 2-{6-[(2-ethyl-5,7-dimethyl-3H-imidazo[4,5-b]pyridin-3-yl)methyl]quinolin-2-yl}aniline is allowed to react with trifluoromethanesulfonic anhydride to obtain N-{2-{6-[(2-ethyl-5,7-dimethyl-3H-imidazo[4,5-b]pyridin-3-yl)methyl]quinolin-2-yl}phenyl}trifluoromethanesulfonamide. Next, the chlorinated solvent solution containing this product is subjected to a post-treatment to eliminate the insoluble materials, by-products, impurities, and the like therefrom. Thus a chlorinated solvent solution containing the product is obtained. Subsequently, an alcohol is added to this solution optionally followed by concentration. Then the crystals of the target product are precipitated from the chlorinated solvent/alcohol mixture. The crystals thus formed are separated from the chlorinated solvent/alcohol mixture to obtain the crystals of the present invention.

As the work-up, citation may be made of, for example, the separation of the insoluble materials by filtration and the like and the elimination of the water-soluble by-products and impurities by washing with an aqueous medium such as water. It is preferred that the aqueous layer is first separated by adding a dilute acid (e.g., dilute acetic acid, and the like) and then the organic layer is subjected to a work-up of washing with water or an aqueous solution of an alkali (e.g., saturated aqueous solution of sodium bicarbonate, and the like).

The crystals of N-[(quinolin-2-yl)phenyl]sulfonamides and salts thereof according to the present invention are those wherein the whole compound is substantially in the crystalline state. The expression "the whole compound is substantially in the crystalline state" means that the crystallization would not proceed any more by usual operations and the physical stability of the crystals as a medicinal compound has been satisfied.

The above meaning will be now illustrated with respect of N-{2-{6-[(2-ethyl-5,7-dimethyl-3H-imidazo[4,5-b]pyridin-3-yl)methyl]quinolin-2-yl}phenyl}trifluoromethanesulfonamide or a salt thereof.

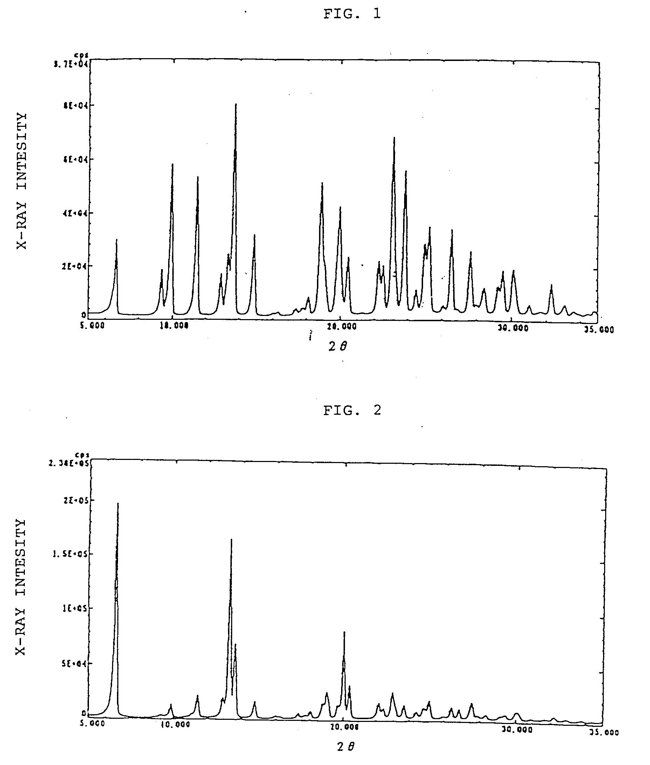

Fig. 1 shows the X-ray diffraction spectrum of the crystals obtained from the ethyl acetate/ethanol system, while Table 1 shows the X-ray diffraction characteristics (diffraction pattern) of the same.

Fig. 2 shows the X-ray diffraction spectrum of the crystals obtained from the toluene/ethanol system. These spectra indicate that these crystals are essentially the same as each other in the crystal form.

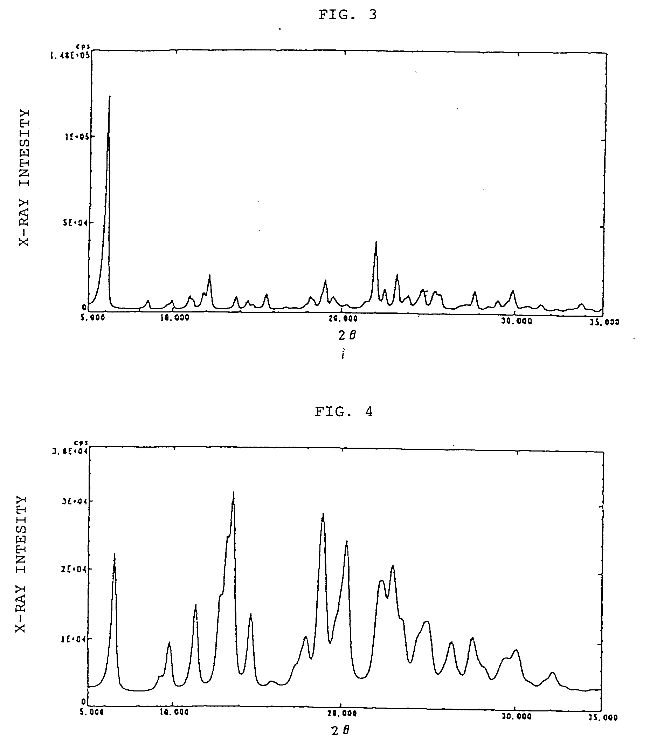

Fig. 3 shows the X-ray diffraction spectrum of the crystals of the hydrochloride obtained from the hydrogen chloride/ethanol system. These crystals are different in the X-ray diffraction spectrum from the ones obtained from the ethyl acetate/ethanol system or the toluene/ethanol system, which indicates the difference in the crystal form. These crystals of Figs. 1 to 3 are highly stable without showing any change in the crystal form under severe conditions (a high temperature, a high humidity, and the like).

In contrast, Fig. 4 shows the X-ray diffraction spectrum of a solid obtained by spray-drying. Although the product obtained by spray-drying is inherently in the form of an amorphous solid, Fig. 4 indicates that this product is not completely amorphous but has somewhat crystallinity. This product undergoes further crystallization under some storage conditions. That is to say, this amorphous product has a poor stability.

The X-ray analysis is carried out by using the following apparatus.
Apparatus: Rotor Flex RU-200 manufactured by Rigaku Denki.
Measurement conditions:
- pipe ball: : Cu
- pipe voltage: : 50 kV
- pipe current: : 200 mA
- sampling width: : 0.010°
- scanning rate: : 0.500°/min,
- scanning axis: : 2θ/θ
- divergent slit: : 1°
- scattering slit: : 1°
- receiving slit: : 0.30 mm

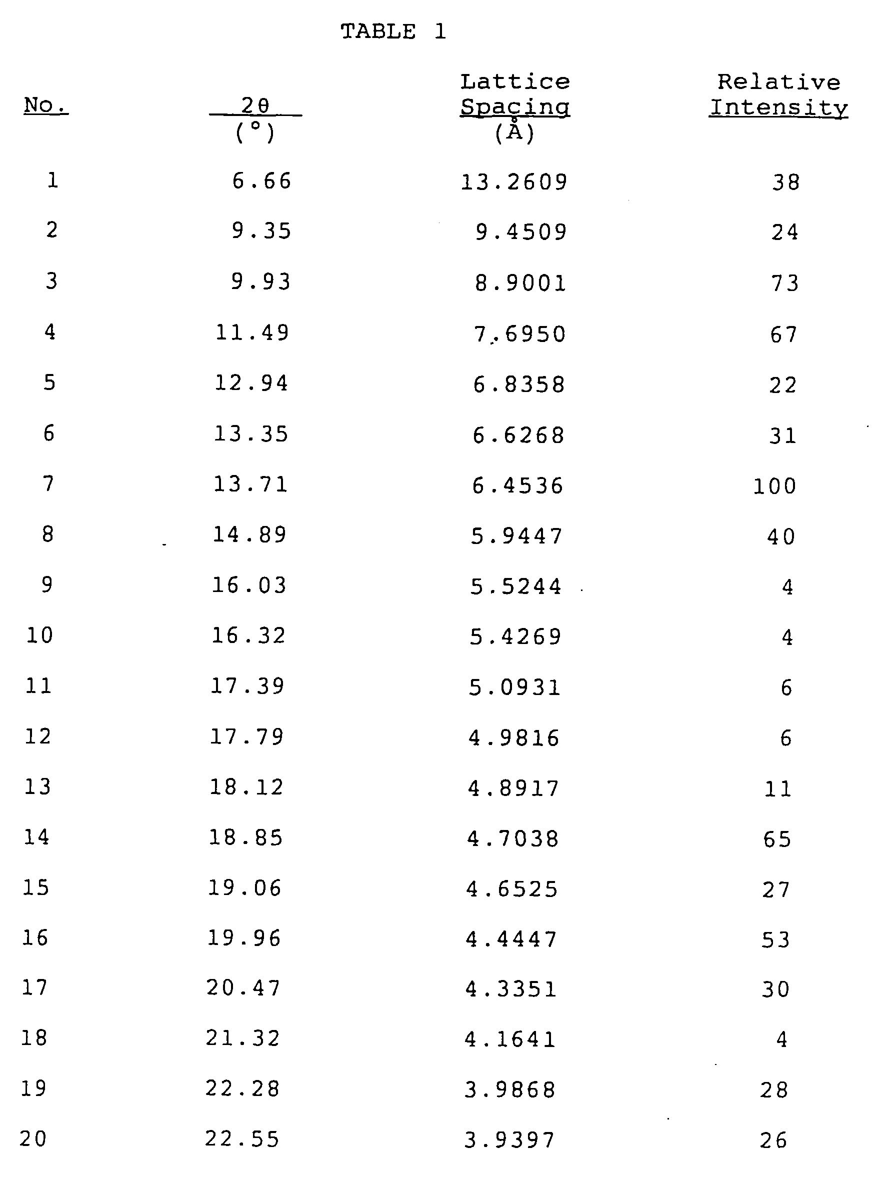

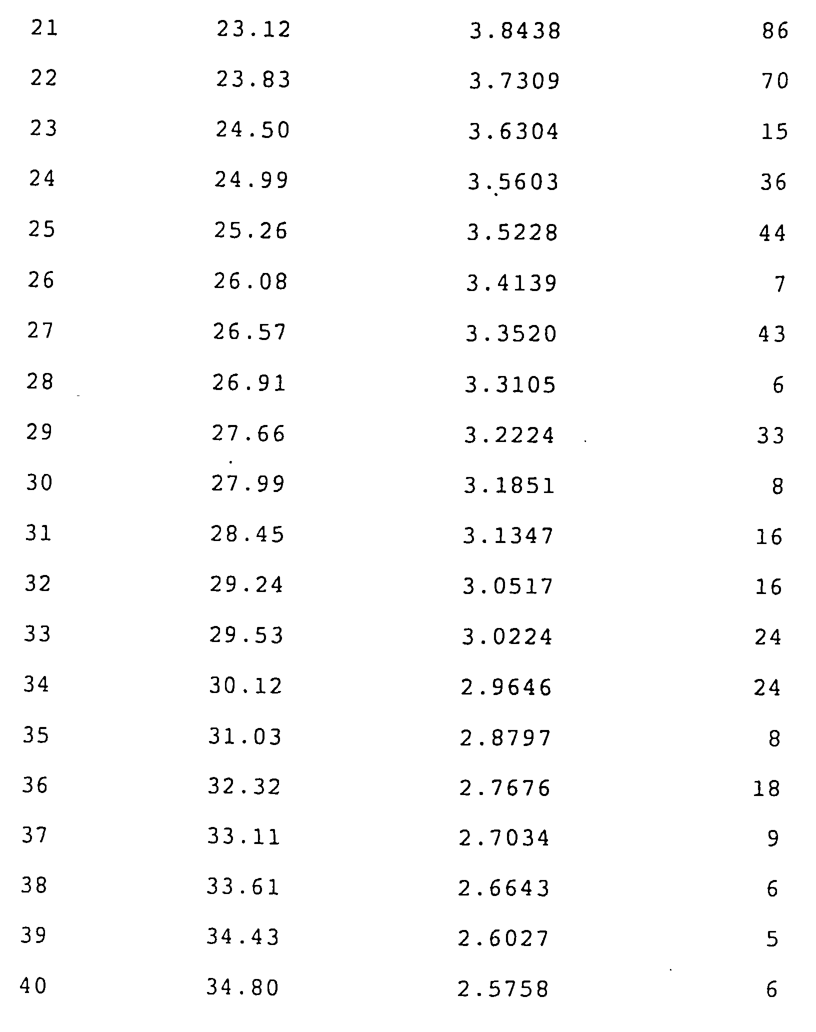

The crystals of the N-[(quinolin-2-yl)phenyl]sulfonamides and salts thereof according to the present invention are highly stable. By the process of the present invention, moreover, highly pure crystals of N-[(quinolin-2-yl)phenyl]sulfonamides and salts thereof can be produced very efficiently and easily.

The present invention will now be illustrated in greater detail with reference to Examples, but it should be understood that the invention is not construed as being limited thereto.

### REFERENCE EXAMPLE 1

### Preparation of solution of crude N-{2-{6-[(2-ethyl-5,7-dimethyl-3H-imidazo[4,5-b]pyridin-3-yl)methyl]quinolin-2-yl}phenyl}trifluoromethanesulfonamide in chloroform:

2-{6-[(2-Ethyl-5,7-dimethyl-3H-imidazo[4,5-b]pyridin-3-yl)methyl]quinolin-2-yl}aniline (8.73 g, 21.4 mmol), triethylamine (6.0 ml, 42.8 mmol) and chloroform (87 ml) were mixed with stirring. To the obtained mixture was added dropwise a solution of trifluoromethanesulfonic anhydride (3.6 ml, 21.4 mmol) in chloroform (36 ml) over 50 minutes under cooling in an ice-bath. After stirring for 10 minutes, 2 M acetic acid (123 ml) was added dropwise into the mixture under ice-cooling. The resulting mixture was separated and the organic layer was washed successively with deionized water (123 ml) twice, a 2% aqueous solution of sodium bicarbonate (123 ml) once and deionized water (123 ml) once to obtain a solution of crude N-{2-{6-[(2-ethyl-5,7-dimethyl-3H-imidazo[4,5-b]pyridin-3-yl)methyl]quinolin-2-yl}phenyl}trifluoromethanesulfonamide in chloroform.

### REFERENCE EXAMPLE 2

### Preparation of solution of crude N-{2-{6-[(2-ethyl-5,7-dimethyl-3H-imidazo[4,5-b]pyridin-3-yl)methyl]quinolin-2-yl}phenyl}trifluoromethanesulfonamide in dichloromethane:

2-{6-[(2-Ethyl-5,7-dimethyl-3H-imidazo[4,5-b]pyridin-3-yl)methyl]quinolin-2-yl}aniline (2.92 g, 7.17 mmol), triethylamine (2.0 ml, 14.3 mmol) and dichloromethane (15 ml) were mixed with stirring. To the obtained mixture was added dropwise a solution of trifluoromethanesulfonic anhydride (1.2 ml, 7.17 mmol) in dichloromethane (12 ml) over 15 minutes under cooling in an ice-bath. After allowing to stand at room temperature for 1 hour, the mixture was ice-cooled again and dichloromethane (15 ml) and a solution of trifluoromethanesulfonic anhydride (0.6 ml, 3.59 mmol) in dichloromethane (6 ml) were added dropwise thereto. Then the mixture was stirred at room temperature for 30 minutes and ice-cooled followed by the addition of 2 M acetic acid (30 ml). The resulting mixture was separated and the organic layer was washed with deionized water (30 ml) twice to obtain a solution of crude N-{2-{6-[(2-ethyl-5,7-dimethyl-3H-imidazo[4,5-b]pyridin-3-yl)methyl]quinolin-2-yl}phenyl}trifluoromethanesulfonamide in dichloromethane.

### EXAMPLE 1

### Production of crystal of N-{2-{6-[(2-ethyl-5,7-dimethyl-3H-imidazo[4,5-b]pyridin-3-yl)methyl]quinolin-2-yl}phenyl}trifluoromethanesulfonamide:

Ethanol (60 ml) was added to the solution of crude N-{2-{6-[(2-ethyl-5,7-dimethyl-3H-imidazo[4,5-b]pyridin-3-yl)methyl]quinolin-2-yl}phenyl}trifluoromethanesulfonamide in chloroform obtained in Reference Example 1 and the mixture was heated to a bath-temperature of 90 to 115°C. After distilling off 176 ml of the solvent, the residue was allowed to stand at 0°C overnight and the crystal material thus precipitated were collected by filtration. After washing with ethanol (10 ml) thrice, the crystals were dried under reduced pressure. A 8.36 g portion of the obtained crystals (8.48 g in total) was taken up and ethyl acetate (425 ml) and ethanol (175 ml) were added thereto. After distilling off 325 ml of the solvent, the residue was allowed to stand at room temperature. The crystals thus precipitated were collected by filtration, washed with ethanol (10 ml) thrice and dried under reduced pressure to obtain 6.36 g of crystals. This recrystallization procedure was carried out once again to obtain 5.52 g of yellow crystals of N-{2-{6-[(2-ethyl-5,7-dimethyl-3H-imidazo[4,5-b]pyridin-3-yl)methyl]quinolin-2-yl}phenyl}trifluoromethanesulfonamide. Fig. 1 shows the X-ray diffraction spectrum of these crystals. m.p.: 252.0°C
- ¹H-NMR (400 MHz, CDCl₃): δ(ppm): 8.22 (d, J=9.2 Hz, 1H); 7.99-8.05 (m, 3H); 7.84 (d, J=8.0 Hz, 1H); 7.69 (dd, J=8.6, 1.4 Hz, 1H); 7.48 (t, J=7.2 Hz, 1H); 7.43 (s, 1H); 7.29 (t, J=8.0 Hz, 1H); 6.94 (s, 1H); 5.67 (s, 2H); 2.82 (q, J=7.6 Hz, 2H); 2.67 (s, 3H); 2.60 (s, 3H); 1.34 (t, J=7.6 Hz, 3H).
- ¹⁹F-NMR (400 MHz, CDCl₃): δ(ppm): -77.183

The residual solvents were determined by gas chromatography [column: PEG 6000 20% Gasprot AS 60/80, glass column manufactured by GL Science, 3 mm (inner diameter) × 3 m, carrier gas: helium, column temperature: 80-160°C (elevated), detection: FID]. Thus it was found out that the contents of the residual chloroform, ethyl acetate and ethanol were not more than 0.01% by weight, 0.07% by weight and 0.04% by weight, respectively.

### EXAMPLE 2

### Production of crystal of N-{2-{6-[(2-ethyl-5,7-dimethyl-3H-imidazo[4,5-b]pyridin-3-yl)methyl]quinolin-2-yl}phenyl}trifluoromethanesulfonamide:

Ethanol (15 ml) was added to the solution of crude N-{2-{6-[(2-ethyl-5,7-dimethyl-3H-imidazo[4,5-b]pyridin-3-yl)methyl]quinolin-2-yl}phenyl}trifluoromethanesulfonamide in dichloroethane obtained in Reference Example 2 and the mixture was concentrated in an evaporator to distill off 40 ml of the solvent. The solid material thus precipitated was collected by filtration and washed with ethanol (5 ml) thrice. Then it was dried under reduced pressure to obtain 2.05 g of N-{2-{6-[(2-ethyl-5,7-dimethyl-3H-imidazo[4,5-b]pyridin-3-yl)methyl]quinolin-2-yl}phenyl}trifluoromethanesulfonamide. A 0.500 g portion of this product was taken up and ethanol (7.5 ml) and ethyl acetate (20 ml) were added thereto. After distilling off 21.5 ml of the solvent by heating, the residue was allowed to stand at 0°C. The crystals thus precipitated were collected by filtration, washed with ethanol (2.5 ml) twice and dried under reduced pressure to obtain 0.415 g of crystals of N-{2-{6-[(2-ethyl-5,7-dimethyl-3H-imidazo[4,5-b]pyridin-3-yl)methyl]quinolin-2-yl}phenyl}trifluoromethanesulfonamide.

### EXAMPLE 3

### Production of crystal of N-{2-{6-[(2-ethyl-5,7-dimethyl-3H-imidazo[4,5-b]pyridin-3-yl)methyl]quinolin-2-yl}phenyl}trifluoromethanesulfonamide:

A 0.500 g portion of the N-{2-{6-[(2-ethyl-5,7-dimethyl-3H-imidazo[4,5-b]pyridin-3-yl)methyl ]quinolin-2-yl}phenyl}trifluoromethanesulfonamide (2.05 g in total) obtained in Example 2 was taken up and toluene (5 ml) and ethanol (20 ml) were added thereto. After distilling off 16 ml of the solvent by heating, the residue was cooled to 0°C. The crystals thus precipitated were collected by filtration, washed with ethanol (2.5 ml) twice and dried under reduced pressure to obtain crystals of N-{2-{6-[(2-ethyl-5,7-dimethyl-3H-imidazo[4,5-b]pyridin-3-yl)methyl]quinolin-2-yl}phenyl}trifluoromethanesulfonamide (0.418 g). Fig. 2 shows the X-ray diffraction spectrum of these crystals.

### EXAMPLE 4

### Production of crystal of N-{2-{6-[(2-ethyl-5,7-dimethyl-3H-imidazo[4,5-b]pyridin-3-yl)methyl]quinolin-2-yl}phenyl}trifluoromethanesulfonamide hydrochloride:

A 0.500 g portion of the N-{2-{6-[(2-ethyl-5,7-dimethyl-3H-imidazo[4,5-b]pyridin-3-yl)methyl]quinolin-2-yl}phenyl}trifluoromethanesulfonamide (2.05 g in total) obtained in Example 2 was taken up and ethanol (3.0 ml) and a solution of hydrogen chloride in ethanol (22% by weight, 0.215 ml) were added thereto. After dissolving by heating, the mixture was cooled to 0°C. The crystals thus precipitated were collected by filtration, washed with ethanol (1.24 ml) 4 times and dried under reduced pressure to obtain crystals of N-{2-{6-[(2-ethyl-5,7-dimethyl-3H-imidazo[4,5-b]pyridin-3-yl)methyl]quinolin-2-yl}phenyl}trifluoromethanesulfonamide hydrochloride (0.376 g). Fig. 3 shows the X-ray diffraction spectrum of these crystals.
- ¹H-NMR (400 MHz, CDCl₃): δ(ppm): 8.28 (d, J=9.2 Hz, 1H); 8.10 (dd, J=8.4, 8.4 Hz, 2H); 8.01 (d, J=7.6 Hz, 1H); 7.84 (d, J=8.0 Hz, 1H); 7.73 (d, J=8.0 Hz, 1H); 7.50 (s, 1H); 7.49 (d, J=8.0 Hz, 1H); 7.32 (dd, J=7.2, 7.6 Hz, 1H); 7.21 (s, 1H); 5.85 (s, 2H); 3.28 (m, 2H); 2.88 (s, 3H); 2.69 (s, 3H); 1.49 (t, J=7.6 Hz, 3H)
- ¹⁹F-NMR (400 MHz, CDCl₃): δ(ppm): -77.121

### REFERENCE EXAMPLE 3

### Production of N-{2-{6-[(2-ethyl-5,7-dimethyl-3H-imidazo[4,5-b]pyridin-3-yl)methyl]quinolin-2-yl}phenyl}trifluoromethanesulfonamide by conventional method:

2-{6-[(2-Ethyl-5,7-dimethyl-3H-imidazo[4,5-b]pyridin-3-yl)methyl]quinolin-2-yl}aniline (5.4 g, 13.3 mmol), triethylamine (27.7 ml, 199 mmol) and dichloromethane (200 ml) were mixed together with stirring. Under cooling at -78°C, trifluoromethanesulfonic anhydride (4.46 ml, 26.5 mmol) was added dropwise thereto. After stirring at room temperature for 1 hour, 2 M acetic acid (130 ml) was added thereto. The resulting mixture was separated and the organic layer was washed with deionized water (100 ml) twice and dried over magnesium sulfate. Then it was filtered, concentrated and purified twice by silica gel column chromatography by using toluene/ethyl acetate as an eluent. However, the purity could not be elevated any more. After purifying by using chloroform/ethyl acetate as an eluent thrice, it was dried at 100°C under reduced pressure overnight to obtain 1.3 g of N-{2-{6-[(2-ethyl-5,7-dimethyl-3H-imidazo[4,5-b]pyridin-3-yl)methyl]quinolin-2-yl}phenyl}trifluoromethanesulfonamide. When the residual solvents were determined by gas chromatography in the same manner as the one described in Example 1, 1.4% of chloroform was detected.

### REFERENCE EXAMPLE 4

### Production of N-{2-{6-[(2-ethyl-5,7-dimethyl-3H-imidazo[4,5-b]pyridin-3-yl)methyl]quinolin-2-yl}phenyl}trifluoromethanesulfonamide by spray drying method:

The crystals (2.5 g), which had been obtained from ethyl acetate/ethanol by the same method as the one described in Example 1, were dissolved in chloroform (150 ml) and ethanol (100 ml) and spray-dried. Fig. 4 shows the X-ray diffraction spectrum of this product.

While the invention has been described in detail and with reference to specific examples thereof, it will be apparent to one skilled in the art that various changes and modifications can be made therein without departing from the spirit and scope thereof.

This application is based on application No. Hei 8-152464 filed in Japan, the entire content of which is incorporated hereinto by reference.

## Claims

1. A crystal of an N-[(quinolin-2-yl)phenyl]sulfonamide represented by the following formula (1) or a crystal of a salt thereof: 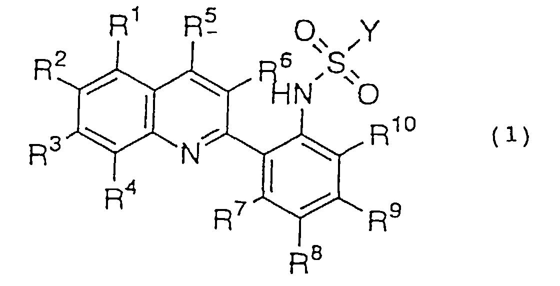
wherein R¹, R³, R⁴, R⁵, R⁶, R⁷, R⁸, R⁹ and R¹⁰ each independently represents a hydrogen atom, a halogen atom, a lower alkyl group, a cyclo lower alkyl group, an aryl group, an aralkyl group, an alkoxy group or -CₘF₂ₘ₊₁;
R² represents a hydrogen atom, a halogen atom, a lower alkyl group, a cyclo lower alkyl group, an aryl group, an aralkyl group, an alkoxy group, -CₙF₂ₙ₊₁ or -CH₂Q, in which Q represents a halogen atom or a monovalent organic group bonded to the carbon atom of -CH₂Q via a nitrogen atom of Q;
Y represents -CₓF₂ₓ₊₁ or an aryl group; and
m, n and x each independently represents an integer of from 1 to 6.

2. A crystal of N-{2-{6-[(2-ethyl-5,7-dimethyl-3H-imidazo[4,5-b]pyridin-3-yl)methyl]quinolin-2-yl}phenyl}trifluoromethanesulfonamide or a crystal of a salt thereof.

3. A crystal of an N-{2-{6-[(2-ethyl-5,7-dimethyl-3H-imidazo[4,5-b]pyridin-3-yl)methyl]quinolin-2-yl}phenyl}trifluoromethanesulfonamide having the X-ray diffraction characteristics as listed in Table 1.

4. A process for producing a crystal of an N-[(quinolin-2-yl)phenyl]sulfonamide or a crystal of a salt thereof which comprises:
crystallizing a crystal of an N-[(quinolin-2-yl)phenyl]sulfonamide represented by the following formula (1) or a crystal of a salt thereof from a solvent solution containing an alcohol: 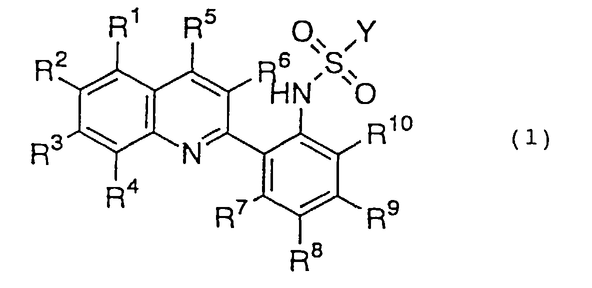
wherein R¹, R³, R⁴, R⁵, R⁶, R⁷, R⁸, R⁹ and R¹⁰ each independently represents a hydrogen atom, a halogen atom, a lower alkyl group, a cyclo lower alkyl group, an aryl group, an aralkyl group, an alkoxy group or -CₘF₂ₘ₊₁;
R² represents a hydrogen atom, a halogen atom, a lower alkyl group, a cyclo lower alkyl group, an aryl group, an aralkyl group, an alkoxy group, -CₙF₂ₙ₊₁ or -CH₂Q, in which Q represents a halogen atom or a monovalent organic group bonded to the carbon atom of -CH₂Q via a nitrogen atom of Q;
Y represents -CₓF₂ₓ₊₁ or an aryl group; and
m, n and x each independently represents an integer of from 1 to 6.

5. The process as claimed in claim 4,
wherein the alcohol is added to a chlorinated solvent solution of a crude of N-[(quinolin-2-yl)phenyl)sulfonamide or a salt thereof to precipitate a crystal; and
the crystal is recrystallized from a solvent solution containing an alcohol.

6. The process as claimed in any one of claims 4 and 5, wherein the N-[(quinolin-2-yl)phenyl]sulfonamide is N-{2-{6-[(2-ethyl-5,7-dimethyl-3H-imidazo[4,5-b]pyridin-3-yl)methyl]quinolin-2-yl}phenyl}trifluoromethanesulfonamide.

7. The process as claimed in any one of claims 4, 5 and 6, wherein the alcohol is ethanol.

8. The process as claimed in any one of claims 5 and 6, wherein the chlorinated solvent is dichloromethane or chloroform.

9. The process as claimed in any one of claims 4, 5 and 6, wherein the solvent solution containing an alcohol is a mixture solvent of an alcohol with ethyl acetate or a mixture solvent of an alcohol with toluene.

10. A process for producing a crystal of N-{2-{6-[(2-ethyl-5,7-dimethyl-3H-imidazo[4,5-b]pyridin-3-yl)methyl] quinolin-2-yl}phenyl}trifluoromethanesulfonamide which comprises:
adding ethanol to a dichloromethane or chloroform solution of a crude of N-{2-{6-[(2-ethyl-5,7-dimethyl-3H-imidazo[4,5-b]pyridin-3-yl)methyl]quinolin-2-yl}phenyl}trifluoromethanesulfonamide or a salt thereof to precipitate a crystal; and
dissolving the crystal in a mixture solvent of ethyl acetate with ethanol or a mixture solvent of toluene with ethanol followed by recrystallization.
